# EUROPEAN PATENT APPLICATION

(11) **EP 3 608 915 A1**
(43) Date of publication of application: **12.02.2020**
(21) Application number: 18187714.3
(22) Date of filing: 07.08.2018
(51) Int. Cl.: G16H 30/40, G16H 40/20, G16H 40/63

(54) **CONTROLLING AN IMAGE PROCESSOR BY INCORPORATING WORKLOAD OF MEDICAL PROFESSIONNALS**

(71) Applicant: Koninklijke Philips N.V., 5656 AE Eindhoven (NL)
(72) Inventor: SEVENSTER, Merlijn, 5656 AE Eindhoven (NL)
(74) Representative: de Haan, Poul Erik

(57) **Abstract**

The invention discloses an apparatus (100) for controlling an image processor. The apparatus comprises a medical image identifier (102) for identifying a medical image to be processed; a workload analyzer (104) for determining a workload of one or more medical professionals; an image processor (106) for performing one or more image processing tasks in respect of the identified medical image; and a controller (108). The controller is configured to determine, based on the determined workload of the one or more medical professionals, whether to use the image processor to perform one or more image processing tasks in respect of the identified medical image.

## Description

### FIELD OF THE INVENTION

The invention relates to controlling an image processor and, more particularly, to controlling an image processor based on a workload of a medical professional.

### BACKGROUND OF THE INVENTION

Automating tasks, for example using techniques involving artificial intelligence, has been proven to improve efficiency in various fields. By introducing automated procedures, such as using a computer to perform tasks rather than a human, tasks can be completed quicker. However, computer-implemented systems for performing tasks that would normally be performed by a skilled and experienced human may not be 100% accurate.

One particular field where the consequences of a task being performed correctly may preclude the implementation of a computer to perform the task is healthcare. For example, radiology involves capturing medical images of subjects and analyzing or processing those medical images to investigate a medical condition, for example by looking for an abnormality in the image that cannot be investigated simply from examining the subject externally. Radiological examinations, which may include medical images, may be processed and analyzed by one or more medical professionals (e.g. a radiologist) whose role it is to interpret the examination and establish some understanding from the radiology examination. While the various tasks involved in analyzing a radiology examination can be performed efficiently by the appropriate medical professional, each task can be particularly time-consuming, thereby taking up valuable time of the medical professional.

Processing radiological examinations constitutes a small part of the overall role of a medical professional, such as a radiologist. As imaging procedures become more readily available, and patients demand more diagnostic certainty, more of a medical professional's time must be committed to analyzing radiological examinations. A consequence of an increased workload is the possibility of fatigue. Fatigue of a medical professional may adversely affect the quality of their work and, in the case of a radiologist, may lead to reduced radiological report quality. In an extreme case, a fatigued radiologist may miss an important feature present in a radiological examination, which could have life-threatening consequences.

Therefore, there is a need for a mechanism that can address some of the above-identified problems. For example, there is a need for an apparatus that can assist a medical professional by performing one or more tasks to reduce the workload of the medical professional.

### SUMMARY OF THE INVENTION

It has been recognized by the inventors of the present disclosure that processing of some radiological examinations (e.g. medical images) may be automated, for example by performing the processing using a computer-implemented image processing technique. Furthermore, it has been recognized that such automated processes may be implemented around the time that a medical professional's workload increases or is particularly high, so as to reduce the chance of the medical professional becoming fatigued.

According to a first aspect, various embodiments provide an apparatus for controlling an image processor, the apparatus comprising: a medical image identifier for identifying a medical image to be processed; a workload analyzer for determining a workload of one or more medical professionals; an image processor for performing one or more image processing tasks in respect of the identified medical image; and a controller configured to: determine, based on the determined workload of the one or more medical professionals, whether to use the image processor to perform one or more image processing tasks in respect of the identified medical image.

In this way, an image processor (e.g. a computer-implemented image processor) may be implemented at an appropriate time, for example when the workload of a medical professional or a team of medical professionals increases by a threshold amount. If the workload of the medical professional does not warrant the need for assistance from the image processor, then the image processor may not be implemented. If the medical professional's workload is particularly high, then relieving them of some of their tasks may help to reduce the chance of the medical professional becoming fatigued and, consequently, this may reduce the risk of errors and mistakes being made by the medical professional.

In some embodiments, the controller may be further configured to, responsive to determining that the image processor is to be used, operate the image processor to perform one or more image processing tasks in respect of the identified medical image based on the determined workload of the one or more medical professionals.

The workload analyzer may be configured to determine the workload of one or more medical professionals based on a decision model.

The workload analyzer may, in some embodiments, be configured to determine the workload of one or more medical professionals based on at least one of: a worklist associated with the one or more medical professionals; an indication of a number of medical professionals available to process a medical image; an indication of a time taken for one or more medical professionals to process a medical image; biometric data acquired from biosensors associated with the one or more medical professionals.

In some embodiments, the identified medical image to be processed may comprise an indication of a radiological specialty relevant to the medical image. The workload analyzer may be configured to determine the workload of one or more medical professionals based on the indication of the radiological specialty.

The image processor may comprise one of: an image processing engine; a predictive model engine; a machine learning engine; an artificial neural network; and a statistical analysis engine.

The image processor may, in some embodiments, be configured to at least one of: detect a defined entity within the medical image; determine a location of a defined entity within the medical image; determine at least one boundary of a defined entity within the medical image; determine at least one dimension of a defined entity within the medical image; determine a volume of a defined entity within the medical image; determine a diagnosis of a defined entity within the medical image; determine prognostic information relating to a defined entity within the medical image; and determine a treatment option for a subject to whom the medical image relates, based on a defined entity within the medical image.

In some embodiments, the controller may be configured to define or adjust a parameter of the image processor based on the determined workload of the one or more medical professionals.

The image processor may be configured to perform one or more image processing tasks in respect of the identified medical image based on a confidence threshold. In some embodiments, the controller may be configured to adjust the confidence threshold based on the determined workload of the one or more medical professionals.

In some embodiments, the apparatus may further comprise a report generator for generating at least part of a report based on an output of the image processor.

The apparatus may, in some embodiments, further comprise a user interface for presenting the generated at least part of a report to a user.

According to a second aspect, various embodiments provide a workstation comprising an apparatus as disclosed herein.

According to a third aspect, various embodiments provide a method of controlling an image processor. The method comprises obtaining an indication of a medical image to be processed; obtaining an indication of a workload of one or more medical professionals; and determining, based on the indication of the workload of the one or more medical professionals, whether to use an image processor to perform one or more image processing tasks in respect the medical image.

The method may, in some embodiments, further comprise, responsive to determining that the image processor is to be used, operating the image processor to perform one or more image processing tasks in respect of the medical image based on the determined workload of the one or more medical professionals.

According to a fourth aspect, various embodiments provide a computer program product comprising a non-transitory computer-readable medium, the computer-readable medium having computer-readable code embodied therein, the computer-readable code being configured such that, on execution by a suitable computer or processor, the computer or processor is caused to perform any of the methods disclosed herein.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified schematic illustration of an example of an apparatus for controlling an image processor according to various embodiments;
Fig. 2 is a simplified schematic illustration of a further example of an apparatus for controlling an image processor according to various embodiments;
Fig. 3 is a simplified schematic illustration of an example of a workstation according to various embodiments;
Fig. 4 is a flowchart of an example of a method of controlling an image processor according to various embodiments;
Fig. 5 is a flowchart of a further example of a method of controlling an image processor according to various embodiments; and
Fig. 6 is a simplified schematic illustration of a processor and a computer-readable medium.

### DETAILED DESCRIPTION OF EMBODIMENTS

Embodiments disclosed herein provide a mechanism by which an automated (e.g. computer-implemented) image processor may, under particular circumstances, be implemented to assist a medical professional performing image processing tasks. For example, a computer-implemented image processor may be used to perform one or more image processing tasks, based on a workload of one or more medical professionals. In some examples, an image processor may be implemented if the workload of a medical professional increases to a level warranting the assistance.

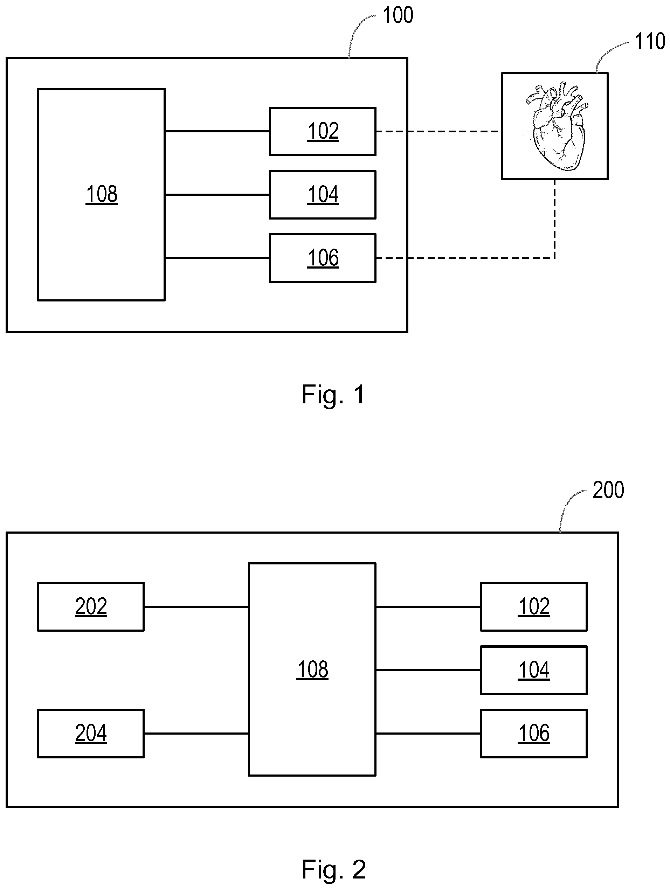

According to various embodiments, Fig. 1 is a simplified schematic illustration of an example of an apparatus 100 for controlling an image processor. The apparatus 100 comprises a medical image identifier 102 for identifying a medical image to be processed. As used herein, the term "processing" is intended to cover any type of image processing and may, in particular, relate to processing for use in medical analysis of the medical image, such as identifying objects in an image that could be indicative of an abnormality or a medical condition. An example of a medical image 110 is shown in Fig. 1. The medical image 110 may form part of a radiological examination (also referred to as a radiological examination study or a medical imaging study). In some embodiments, a radiological examination may comprise just a medical image while, in other embodiments, a radiological examination may comprise one or more medical images in addition to other information, such as text, data included in one or more data fields, annotations or the like. The medical image 110 be acquired using any medical imaging modality or technique including, for example, x-ray imaging, magnetic resonance imaging (MRI), ultrasonography or ultrasound, positron emission tomography (PET), computed tomography (CT) and single-photon emission computed tomography (SPECT). Other imaging modalities that are familiar to those skilled in the art may also be used to acquire the medical image 110.

The medical image 110 may be associated with a subject. As used herein, the term "subject" is intended to refer to any human or animal in respect of whom a medical image 110 can be acquired, for example using one of the above-mentioned techniques. The subject may, for example, comprise a patient of a medical facility in respect of whom a medical image 110 has been acquired, for example for investigative, diagnostic or prognostic purposes.

The medical image 110 to be processed may comprise one of a plurality of medical images that are to be processed (e.g. by having one or more image processing tasks performed in respect of them). The medical image 110, or some name, label or other identification means enabling the medical image to be identified may be included in a list (e.g. a work list). A medical professional or a team of medical professionals may work through the work list, processing each medical image in turn, or according to some defined order of priority.

The medical image identifier 102 may identify the medical image 110 to be processed using the name or identification means of the medical image, and the identified medical image may be provided to the medical professional for processing. For example, a plurality of medical images may be stored in a storage medium of a computing device or in a server. Once a medical image 110 has been identified to be processed, the medical image may be obtained or acquired from the storage medium and provided to be accessible (e.g. via a computing device or workstation) to a user (e.g. a medical professional, such as a radiologist).

In some embodiments, the medical image 110 may be stored with a plurality of other medical images in a Picture Archiving and Communication System (PACS), via which medical images (e.g. radiological examinations) may be shared among medical professionals within a medical facility or shared among multiple medical facilities. Thus, the plurality of medical images (or a plurality of tags or labels identifying the medical images) may be listed in a PACS work list. The work list (e.g. the PACS work list) may be specific to a particular team, or radiological specialty, such as the specialties discussed above. The radiological examinations listed in the work list may include data arranged according to the Digital Imaging and Communications in Medicine (DICOM) standard. For example, each entry in the work list may include a medical image 110 and associated data. A radiological examination may include one or more medical images, and may additionally include data associated with the medical image(s) 110. For example, a radiological examination may include data identifying the subject (e.g. the subject's name, date of birth, patient identification number, medical record number, medical imaging study accession ID, and the like). The radiological examination may, in some embodiments, include an indication of the relevant medical condition (e.g. a medical condition from which the subject is suffering or suspected of suffering, or the medical condition forming the subject of the medical imaging study). In some examples, the radiological examination may include an indication of the imaging modality used to capture the medical image (e.g. x-ray, CT, MRI, and so on). The radiological examination may include an indication of the body part and/or the anatomical object captured in the medical image 110. For example, such indication may include a label identifying the region of the subject's body (e.g. the head, the pelvis, the chest, and so on) from which the medical image 110 was captured.

The apparatus 100 further comprises a workload analyzer 104 for determining a workload of one or more medical professionals. A workload of a medical professional may, for example, comprise an indication or measure of an amount of work the medical professional has done or is expected or scheduled to do in a period of time. In other examples, a medical professional's workload may be defined or estimated using some other metric.

In some examples, the workload analyzer 104 may receive input data from one or more sources. A model may be used to calculate an indication of a medical professional's workload based on some or all of the input data. In one example, the workload analyzer 104 may be configured to determine the workload of one or more medical professionals based on a model, such as a decision model. The model (e.g. the decision model) may, in some embodiments, comprise one or more of an algebraic model, a statistical model, and a predictive model will stop for example, a predictive model may comprise a model developed using machine learning techniques, whereby a model is trained using a set of training data. The workload analyzer 104 may (e.g. using the decision model) output an indication of the workload of a medical professional, or of multiple medical professionals. In some examples, the workload indication may comprise a workload assessment score represented, for example, on a numerical scale. The numerical scale may be from 0 to 1, where 0 indicates a minimum workload and 1 indicates the maximum workload. In other examples, the numerical scale may be represented on a 5-item scale, such as 0 to 4, where 0 indicates a minimum workload and 4 indicates the maximum workload. In other examples, the workload may be indicated in another way, for example as a percentage.

The workload analyzer 104 may, in some embodiments, be configured to determine the workload of one or more medical professionals based on at least one of: a worklist associated with the one or more medical professionals; an indication of a number of medical professionals available to process a medical image; an indication of a time taken for one or more medical professionals to process a medical image; biometric data acquired from biosensors associated with the one or more medical professionals.

A work list associated with a medical professional may, for example, comprise the work list discussed above, from which the medical image 110 to be processed is determined or identified. For example, the work list may comprise a PACS work list. The workload analyzer 104 may obtain and/or analyze such a work list so as to determine from the work list a measure of a medical professional's workload. For example, the workload analyzer 104 may determine when the number of medical images/examinations 110 on the work list exceeds a defined threshold. In other examples, the workload analyzer 104 may determine when the number of medical images/examinations 110 on the work list has increased or decreased, and may determine a measure of the increase or decrease. The change (e.g. the increase or decrease) may be measured for a particular duration of time. For example, the workload analyzer 104 may determine that the number of cases on the work list has increased by 10% in the past hour. In another example, the workload analyzer 104 may determine that the number of cases on the work list tends to increase at a particular time and/or on a particular day (e.g. the number of cases on the work list tends to increase by 10% at around 10am on a Monday morning).

An indication of a number of medical professionals available to process a medical image 110 may be used as an indicator of the workload of one or more medical professionals as this would provide an indication of how many medical professionals (e.g. radiologists) are available to assist with processing medical images waiting to be processed (e.g. in the work list). Such an indication may be obtained by determining the number of medical professionals signed in or logged on to a particular system (e.g. a PACS client). In another example, an indication of the number of available medical professionals may be obtained by determining which medical professionals have processed medical images recently (e.g. in the past hour).

An indication of a time taken for one or more medical professionals to process a medical image 110 may be used in determining the workload of the medical professional(s). For example, if the average medical image processing time for a medical professional is determined to have decreased, then this may be indicative of an increased workload (e.g. if the medical professional is rushing the processing of medical images in order to work through the medical images waiting to be processed.

In some embodiments, biometric data acquired from biosensors associated with the one or more medical professionals may be used to determine workload levels. A biosensor, such as a heart rate monitor, a blood pressure monitor, and the like, may be worn by a medical professional in order to monitor one or more characteristics of the medical professional. A change (e.g. an increase) in a characteristic (e.g. heart rate) of the medical professional beyond a threshold level or by a threshold amount may be indicative of an increased workload of the medical professional. In some examples, one or more biosensors may be incorporated into a wearable device (e.g. a smartwatch) worn by the medical professional.

Any of the indications or measurements identified above may be used by the workload analyzer 104 in determining the workload of the one or more medical professionals. In some examples, a more accurate measure of the workload may be obtained if multiple measurements or indications are used.

The workload analyzer 104 may, in some embodiments, take into account a team, department, radiological specialty and/or area of expertise of a medical professional, also referred to as a radiological section, when determining the workload for that medical professional. In some examples, a medical facility (e.g. a hospital), may comprise radiological specialties including neurological, abdominal, thoracic, musculoskeletal, pediatrics, nuclear medicinal and breast. In other examples, the workload analyzer 104 may take into account a particular type of processing or a particular area of examination relevant to the medical image 110 to be processed. For example, a separate workload may be determined for each team, department or specialty and/or for each type of examination required for a medical image (e.g. general radiological examination or specialist radiological examination, such as neurological, abdominal, and thoracic). In some embodiments, the workload analyzer 104 may determine a workload based on the part of the subject's body, or the anatomical object of which the medical image is captured. More generally, the workload analyzer 104 may determine a workload based on the radiological specialty relevant to the medical image, or the radiological specialty of the medical professional(s) reading or analyzing the medical image/examination. Thus, the identified medical image to be processed may comprise an indication of a radiological specialty relevant to the medical image. The workload analyzer 104 may be configured to determine the workload of one or more medical professionals based on the indication of the radiological specialty. In this way, the workload of a particular subset of medical professionals may be determined so that the workloads of just the relevant medical professionals (e.g. those medical professionals having the relevant radiological specialty) are taken into account.

The apparatus 100 further comprises an image processor 106 for performing one or more image processing tasks in respect of the identified medical image 110. The apparatus 100 may, in some embodiments, comprise a plurality of image processors 106. Each image processor 106 may be configured to perform at least one image processing task in respect of the medical image 110. The image processor 106 or image processors may, for example, comprise circuitry or an engine configured to perform one or more image processing tasks. In some examples, an image processor 106 may comprise one of: an artificial intelligence engine; an image processing engine; a predictive model engine; a machine learning engine; an artificial neural network; and a statistical analysis engine. A machine learning engine may employ machine learning techniques including, for example, decision tree algorithms, artificial neural networks, deep learning neural networks, support vector machines, Bayesian networks, and the like, which will be familiar to those skilled in the field of machine learning.

The image processor 106 may, for example, be configured to automatically process radiological image data (e.g. the medical image 110), and provide an output based on the particular image processing task performed.

The image processor 106 may be configured to perform any image processing task, particular, an image processing task relevant to the field of medical image analysis. In some examples, an image processor 106 may be configured to detect the presence of a defined entity in the medical image 110. For example, an image processor may be configured to detect the presence of a lesion, a tumor, an abnormality, a fracture, a tear, or some other feature visible or detectable in the medical image 110. In some examples, an image processor 106 may be configured to locate or determine the location of (e.g. identify the location, for example using a coordinate system) a defined entity (e.g. a lesion, tumor, abnormality etc.) in the medical image. An image processor 106 may, in some examples, be configured to determine at least one the boundary of a defined entity or of a detected entity. For example, once a defined entity has been detected by one of the image processors 106, an image processor (e.g. the same image processor that performed the detection or a different one of the image processors) may locate and/or detect one or more boundaries of the defined entity. In some examples, an image processor 106 may determine at least one size, dimension or volume of a defined entity or of a detected entity. For example, a dimension may include a length, a width, a height or a depth. This may be referred to as quantification of the defined entity. A volume of the defined entity may be determined or estimated using one or more of the determined dimensions and/or one or more other methods. In some examples, an image processor 106 may be configured to perform a segmentation task (e.g. lesion segmentation) in respect of a defined entity in the medical image. For example, the defined entity (e.g. a lesion) may be segmented, delineated or visually separated from other parts of the medical image. An image processor 106 may, in some examples, be configured to determine a change in one or more characteristics or features of the medical image over time (e.g. as compared to a previously-captured medical image). An image processor 106 may be configured to determine a diagnosis of a defined entity. For example, the image processor 106 may be configured to recognize or determine a feature present in the medical image that is representative of a particular medical condition. In making such a determination, an output of the image processor 106 may be combined with information obtained from another source, such as an electronic health record associated with the subject. In some embodiments, an image processor 106 may be configured to determine a treatment option based on a detected entity. Such a determination may, for example, be made using a lookup table or database, based on the nature of the defined entity, and/or using a predictive model (e.g. a machine learning model).

In some examples, an image processor 106 may be configured to perform an image processing task in respect of the medical image 110 based on one or more parameters or variables. The parameters or variables may be configured automatically (e.g. via a processor) or manually by a user (e.g. a medical professional). One such parameter may comprise a number of determinations that the image processor 106 is to make. For example, an image processor 106 may be configured to make just one determination (e.g. detect a defined entity) based on the processing of the medical image 110. In other examples, however, an image processor 106 may be configured to make multiple determinations (e.g. detect a defined entity, characterize or determine the nature of the defined entity, determine a volume of the defined entity, and determine a suitable treatment option for the subject) based on the processing of the medical image 110. The amount of processing (e.g. the number processing tasks) to be performed by the image processor 106 or by multiple image processors may be selected by adjusting the relevant variable of the image processor. In one example, an image processor 106 may be configured to analyze or search the medical image 110 for one or more defined entities, such as edema, fractures, nodules, unexpected mass, pleural effusion, and the like.

Another parameter of an image processor 106 that may be configured (e.g. automatically or manually) comprises a confidence threshold of the image processor, also referred to as a sensitivity threshold or level. The confidence threshold of an image processor 106 may be configured and/or adjusted so that the image processor provides an output only if the determination made by the image processor meets or exceeds a defined threshold (e.g. a confidence threshold). An image processor 106 operating at a relatively lower confidence threshold, could produce a finding (e.g. an output) when there is less evidence as compared to an image processor operating at a relatively higher confidence threshold. For example, an image processor 106 operating with a confidence threshold set to 90% would be less likely to provide an output (e.g. completing a processing task that it is configured to perform) than if the confidence threshold were set to 60%. However, with the confidence threshold set to 90%, there is a smaller chance that the output may be inaccurate (e.g. a false positive) than if the confidence threshold were set to 60%.

The apparatus 100 further comprises a controller 108. The controller 108 may be operatively coupled to the medical image identifier 102, the workload analyzer 104 and/or the image processor 106. Thus, the controller 108 may, in some embodiments, control one or more of the elements of the apparatus 100 to which it is operatively coupled. The controller 108 may comprise one or more processors or processing circuitry, and may be configured to execute a set of instructions stored, for example, on a storage medium accessible by the processor/controller.

The controller 108 is configured to determine, based on the determined workload of the one or more medical professionals, whether to use the image processor 106 to perform one or more image processing tasks in respect of the identified medical image 110. The controller may, in some embodiments, use a reasoning module, one or more rules, or one or more look up-tables to interpret the output of the workload analyzer 104. For example, the controller 108 may convert a numerical output (e.g. a workload assessment score) from the workload analyzer 104 into a variable specification or instruction for the image processor 106. In a general example, if the output of the workload analyzer 104 indicates that the workload of a medical professional (or of a plurality of medical professionals) is above a threshold level, then the controller 108 may determine that an image processor 106 should be used to perform one or more image processing tasks in respect of the medical image 110. In other words, if it is determined that a medical professional is becoming too busy, or as to any medical images to process, there is a risk that the quality of processing by the medical professional may reduce and, therefore, the controller 108 may implement an image processor 106 to relieve the medical professional of some of the burden of the processing tasks. Thus, the controller 108 may be configured to implement the image processor 106 responsive to determining that the workload of the one or more medical professionals is above a defined threshold level.

In some examples, if the workload analyzer 104 determines that the workload of a medical professional is below a defined threshold level, then the controller 108 may determine that assistance from the image processor 106 is not required and, therefore, an image processor may not be implemented. On the other hand the controller 108 may be further configured, responsive to determining that the image processor 106 is to be used, to operate the image processor to perform one or more image processing tasks in respect of the identified medical image 110 based on the determined workload of the one or more medical professionals. Thus, the controller 108 may automatically implement the image processor 106 and/or operate the image processor to perform one or more image processing tasks if it is determined that the workload exceeds a threshold level. In other embodiments, however, if it is determined that the workload exceeds a threshold level, then the controller 108 may generate an alert or generate an indication for delivery to a user (e.g. a medical professional) so that the user can make a decision as to whether or not the image processor 106 should be implemented.

In some embodiments, the controller 108 may further determine the particular image processing task to be performed by the image processor 106. In embodiments in which the apparatus includes a plurality of image processors 106, the controller 108 may determine which of the plurality of image processors is to be implemented, and which task or tasks the selected image processor(s) should perform in respect of the medical image 110.

Once an image processor 106 has been implemented, its use may continue (e.g. the image processor may be used to perform a particular image processing task in respect of each medical image to be processed) until a further instruction is received by the controller 108, until the controller determines that the image processor 106 is no longer required, or until the controller determines that implementation of a different or additional image processor is required. The implementation of an image processor 106 by the controller 108 may be considered to be dynamic, in that the determination of whether or not to use an image processor 106, and the determination of which image processor(s) to use may change responsive to the workload of the medical professional changing. Thus, the workload analyzer 104 may constantly or repeatedly determine the workload of a medical professional, and the decision to use or not to use an image processor 106 to assist the medical professional may be updated continuously or intermittently.

In some embodiments, the controller 108 may be configured to implement the image processor 106 responsive to determining that the workload of the one or more medical professionals has increased by a threshold amount. Thus, an unexpected or unaccounted for increase in the workload of a medical professional may prompt the controller 108 to implement an image processor 106 or multiple image processors to assist medical professional. In some examples, any change in workload may be measured over a defined period of time. Thus, the controller 108 may be configured to implement the image processor 106 responsive to determining that the workload of the one or more medical professionals has increased by a threshold amount in a defined period of time. For example, if the workload of a medical professional is determined to have increased by 10% in the space of one hour, then this may be indicative that the medical professional could benefit from assistance from a computer implemented image processor 106 and, as such, the controller 108 may implement an image processor.

If an image processor 106 has been implemented (e.g. based on the determined workload of one or more medical professionals), then the controller 108 may adapt the operation of the image processor in response to changes in the determined workload. Thus, in some examples, the controller 108 may be configured to define or adjust a parameter of the image processor 106 based on the determined workload of the one or more medical professionals. For example, if an output of the workload analyzer 104 indicates that the workload of a medical professional has increased from "medium" to "medium-high", then the controller 108 may reduce the confidence threshold of the image processor 106 and/or may increase the number of determinations to be made by the image processor. In this way, more use is made of the image processor 106, and the image processor is likely to generate a greater number of outputs (e.g. due to the confidence threshold), thereby reducing the burden of the medical professional. Thus, as noted above, the image processor 106 may be configured to perform one or more image processing tasks in respect of the identified medical image 110 based on a confidence threshold. The controller 108 may be configured to adjust the confidence threshold based on the determined workload of the one or more medical professionals. In other examples, the controller 108 may be configured to define or adjust another parameter of the image processor 106 based on the determined workload of the one or more medical professionals.

Fig. 2 is a simplified schematic of a further example of an apparatus 200 for controlling an image processor. The apparatus 200 may comprise the medical image identifier 102, the workload analyzer 104, the image processor 106 and/or the controller 108 as disclosed herein. In some embodiments, the apparatus 200 may further comprise a report generator 202 for generating at least part of a report based on an output of the image processor 106. For example, if an image processor 106 detects a defined entity in the medical image 110, then an indication that the defined entity has been detected may be generated for inclusion in a report. In some embodiments, the report generator 202 may generate just a portion (a section, a paragraph, or a unit of text) of a report while, in other embodiments, the report generator 202 may generate an entire report.

The report generator 202 may be configured to generate the report or a portion of a report in a computer-readable format, such as XML or JSON. In other examples, the report generator 202 may be configured to generate the report or a portion of the report in a human-readable format. The report generator 202 may, in some embodiments, be configured to convert the output of the image processor 106 into a human-readable format. In some embodiments, the apparatus 100, 200 or the report generated 202 may include a format conversion unit to convert a computer-readable report (or portion thereof) into a human-readable format. Such a conversion may be made based on one or more rules. In one example, the image processor 106 may generate an output indicating that edema is detected in a chest x-ray with a certainty of 80%. In this example, the report generator 202 may convert this finding into text suitable for report, such as a sentence indicating that "edema has been detected with high certainty".

The apparatus 200 may, in some embodiments, further comprise a user interface 204 for presenting the generated at least part of a report to a user. For example, a report or a portion of a report generated by the report generator 202 may be presented to a user via the user interface 204. In some examples, the user interface 204 may present information (e.g. the report) in a user-editable manner, such that user is able to modify the report. In some examples any report generated by the report generator 202 may be presented for review by a medical professional, since the output of the user interface 106 alone may not be sufficiently reliable.

An illustrative example of how the apparatus 100, 200 may be used is described below. The workload analyzer 104 may determine that, at around 1 1am, a medical professional experiences an increased workload, from a "normal" level to a "medium-high" level. The medical image identifier 102 may identify that a medical image 110 is to be processed. In view of the increased workload, the controller 108 may determine that an image processor 106 should be implemented to perform an image processing task in respect of the medical image 110. In this example, the image processor 106 should perform an interpretation of a chest x-ray, in the thorax section. The image processor 106 may, for example, be configured to determine whether a medical image appears "normal" (e.g. exhibits no signs of an abnormality, or a defined entity, such as a fractured bone in the case of a bone x-ray). Since the workload is only considered medium-high, the parameters of the image processor 106 are set such that the image processor produces an output only for those chest x-rays which are highly likely (e.g. having a confidence level of greater than 99%) to be "normal" (i.e. no abnormalities present). Once the image processor 106 has performed its image processing task in respect of the medical image 110, a report may be generated (e.g. using a report generator 202) and provided for presentation to an interpreting radiologist for further review. The interpreting radiologist may approve the report if he or she agrees with the finding of the image processor 106.

In another example, if the workload were to increase too "high", then the confidence threshold may be reduced, for example from 99% to 95%, such that the image processor 106 processing a medical image 110 if the outcome of the processing is likely (e.g. having a confidence threshold of greater than 95%) to be "normal". Thus, in view of the increased workload, a greater number of medical images are to be processed by the image processor 106. In some examples, if the workload of the medical professional is extremely high, then the apparatus 100, 200 (or the report generator 202) may be configured to generate a final report, which is not passed to a medical professional for review.

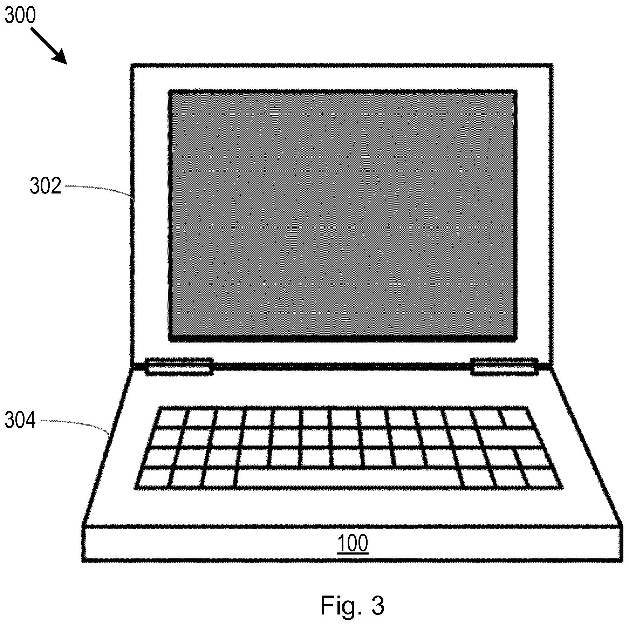

According to a second aspect, various embodiments disclosed herein provide a workstation. In some examples, the apparatus 100, 200 may comprise or be incorporated into a workstation. Fig. 3 is a simplified schematic of an example of a workstation 300. The workstation 300 may comprise the apparatus 100, 200 as disclosed herein. The workstation 300 may also comprise a display screen 302 and/or a user input device 304, such as a keyboard.

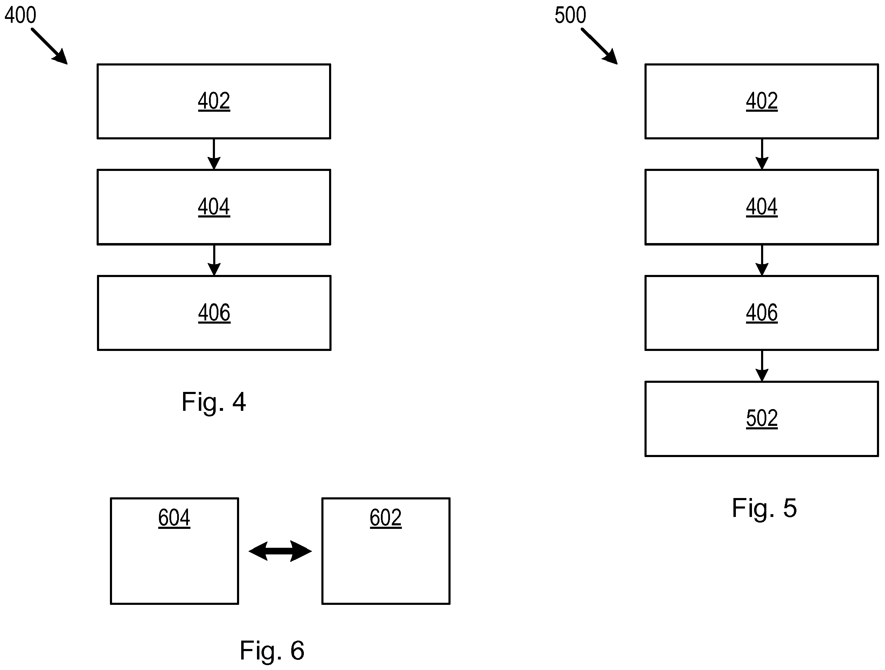

According to a third aspect, various embodiments disclosed herein provide a method for controlling an image processor. Fig. 4 is a flowchart of an example of a method 400 for controlling an image processor (e.g. the image processor 106). The method 400 comprises, at step 402, obtaining an indication of a medical image to be processed. The indication of the medical image to be processed may, for example, be obtained or acquired from a work list, such as a PACS work list, as discussed above. At step 402, the method 400 comprises obtaining an indication of a workload of one or more medical professionals. The indication of the workload may, for example, be obtained from or using the workload analyzer 104, as discussed above. The method 400 comprises, at step 406, determining, based on the indication of the workload of the one or more medical professionals, whether to use an image processor to perform one or more image processing tasks in respect the medical image. For example, if it is determined at step 406 that the workload of the one or more medical professionals meets or exceeds a threshold level, then an image processor may be implemented to perform an image processing task in respect of the medical image, so as to reduce the burden of a medical professional, and ease their workload.

Fig. 5 is a flowchart of a further example of a method 500 for controlling an image processor. The method 500 may comprise one or more steps of the method 400 discussed above. The method 500 may further comprise, at step 502, responsive to determining that the image processor is to be used, operating the image processor to perform one or more image processing tasks in respect of the medical image based on the determined workload of the one or more medical professionals. In some examples, the method may comprise determining which of a plurality of image processors 106 should be implemented, and which image processing tasks should be performed by the selected image processor(s).

The method 400, 500 may, in some embodiments, further comprise generating at least part of a report based on an output of an image processor. In some embodiments, the method 400, 500 may further comprise delivering the at least part of a report for presentation to a user, or presenting the at least part of the report to a user. For example, the report (or portion thereof) may be presented to the user via the user interface 204, as discussed above.

Controlling an image processor using the method 400, 500 as discussed above may help to ease the workload of a medical professional at a time when the medical professional's workload is considered to be particularly high. At such times, a medical professional may begin to suffer from fatigue, which may lead to mistakes or errors being made during a radiological examination. The methods disclosed herein may help to reduce the chance of such errors occurring.

According to a fourth aspect, various embodiments disclosed herein provide a computer program product. Fig. 6 is a simplified schematic of an example of a processor 602 and a computer-readable medium 604. According to some embodiments, a computer program product comprises a non-transitory computer-readable medium 604, the computer-readable medium having computer-readable code embodied therein, the computer-readable code being configured such that, on execution by a suitable computer or processor 602, the computer or processor is caused to perform steps of the methods 400, 500 disclosed herein. In some embodiments, the processor 602 may comprise, function as part of, or operate in association with the controller 108. Similarly, one or more image processors 106 may form part of the processor 602.

The processor 602 can comprise one or more processors, processing units, multi-core processors or modules that are configured or programmed to control elements of the apparatus 100, 200 in the manner described herein. In particular implementations, the processor 602 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus (100) for controlling an image processor, the apparatus comprising:
a medical image identifier (102) for identifying a medical image (110) to be processed;
a workload analyzer (104) for determining a workload of one or more medical professionals;
an image processor (106) for performing one or more image processing tasks in respect of the identified medical image; and
a controller (108) configured to:
determine, based on the determined workload of the one or more medical professionals, whether to use the image processor to perform one or more image processing tasks in respect of the identified medical image.

2. An apparatus (100) according to claim 1, where the controller (108) is further configured to:
responsive to determining that the image processor (106) is to be used, operate the image processor to perform one or more image processing tasks in respect of the identified medical image (10) based on the determined workload of the one or more medical professionals.

3. An apparatus (100) according to claim 1 or claim 2, wherein the workload analyzer (104) is configured to determine the workload of one or more medical professionals based on a decision model.

4. An apparatus (100) according to any of the preceding claims, wherein the workload analyzer (104) is configured to determine the workload of one or more medical professionals based on at least one of: a worklist associated with the one or more medical professionals; an indication of a number of medical professionals available to process a medical image; an indication of a time taken for one or more medical professionals to process a medical image; biometric data acquired from biosensors associated with the one or more medical professionals.

5. An apparatus (100) according to any of the preceding claims, wherein the identified medical image (110) to be processed comprises an indication of a radiological specialty relevant to the medical image; and
wherein the workload analyzer (104) is configured to determine the workload of one or more medical professionals based on the indication of the radiological specialty.

6. An apparatus (100) according to any of the preceding claims, wherein the image processor (108) comprises one of: an image processing engine; a predictive model engine; a machine learning engine; an artificial neural network; and a statistical analysis engine.

7. An apparatus (100) according to any of the preceding claims, wherein the image processor (106) is configured to at least one of: detect a defined entity within the medical image; determine a location of a defined entity within the medical image; determine at least one boundary of a defined entity within the medical image; determine at least one dimension of a defined entity within the medical image; determine a volume of a defined entity within the medical image; determine a diagnosis of a defined entity within the medical image; determine prognostic information relating to a defined entity within the medical image; and determine a treatment option for a subject to whom the medical image relates, based on a defined entity within the medical image.

8. An apparatus (100) according to any of the preceding claims, wherein the controller (108) is configured to define or adjust a parameter of the image processor (106) based on the determined workload of the one or more medical professionals.

9. An apparatus (100) according to any of the preceding claims, wherein the image processor (106) is configured to perform one or more image processing tasks in respect of the identified medical image based on a confidence threshold; and
wherein the controller (108) is configured to adjust the confidence threshold based on the determined workload of the one or more medical professionals.

10. An apparatus (100, 200) according to any of the preceding claims, further comprising:
a report generator (202) for generating at least part of a report based on an output of the image processor (106).

11. An apparatus (100, 200) according to claim 10, further comprising:
a user interface (204) for presenting the generated at least part of a report to a user.

12. A workstation (300) comprising the apparatus (100, 200) of any of the preceding claims.

13. A method (400) of controlling an image processor, the method comprising:
obtaining (402) an indication of a medical image to be processed;
obtaining (404) an indication of a workload of one or more medical professionals; and
determining (406), based on the indication of the workload of the one or more medical professionals, whether to use an image processor to perform one or more image processing tasks in respect the medical image.

14. A method (400, 500) according to claim 13, further comprising:
responsive (502) to determining that the image processor is to be used, operating the image processor to perform one or more image processing tasks in respect of the medical image based on the determined workload of the one or more medical professionals.

15. A computer program product comprising a non-transitory computer-readable medium (604), the computer-readable medium having computer-readable code embodied therein, the computer-readable code being configured such that, on execution by a suitable computer or processor (602), the computer or processor is caused to perform the method of any of claims 13 to 14.
